# EUROPEAN PATENT SPECIFICATION

(11) **EP 2 878 214 B1**
(45) Date of publication and mention of the grant of the patent: **20.09.2017**
(21) Application number: 13823760.7
(22) Date of filing: 22.07.2013
(51) Int. Cl.: A24F 47/00, H02J 7/00, A61M 15/06

(54) **PORTABLE CHARGING DEVICE FOR ELECTRONIC CIGARETTE**
TRAGBARE LADEVORRICHTUNG FÜR ELEKTRONISCHE ZIGARETTE
DISPOSITIF PORTABLE DE CHARGE POUR CIGARETTE ÉLECTRONIQUE

(30) Priority: 26.07.2012 KR 20120081601
(43) Date of publication of application: 03.06.2015
(73) Proprietor: Shin, Jong-Soo, Cheongju-si, Chungbuk 361-270 (KR)
(72) Inventor: Shin, Jong-Soo, Cheongju-si, Chungbuk 361-270 (KR)
(74) Representative: Witte, Weller & Partner Patentanwälte mbB
(86) International application number: PCT/KR2013/006543
(87) International publication number: WO 2014/017794

(56) References cited:
- JP-A- 2011 087 569
- JP-U- 3 164 408
- KR-B1- 101 057 774
- KR-U- 20110 009 632
- KR-Y1- 200 451 581
- US-A1- 2009 283 103

## Description

### Technical Field

The present invention relates to a charging device for an electronic cigarette that is used to charge a battery supplying electricity to an electronic cigarette. More specifically, the present invention relates to a portable charging device for an electronic cigarette, which includes: a main body having a first seat part electrically connected to a battery of an electronic cigarette, and a second seat part electrically connected to a smoke generating part of the electronic cigarette; a cap detachably attached to the main body and covering an inhaling bit of the smoke generating part, and sterilizing the inhaling bit; and an operation unit electrically connected to the first seat part, the second seat part, and the cap, and controlling the charging of the battery, the operation of the smoke generating part, and the sterilization of the electronic cigarette, and which can easily generate smoke while charging the battery of the electronic cigarette and can sterilize the electronic cigarette during a process of charging the battery of an electronic cigarette.

### Background Art

An electronic cigarette is proposed and used as a substitute for typical cigarettes, such as cigarettes, cigars and pipe tobacco. The electronic cigarette has a shape similar to that of a typical cigarette and generates smoke by vaporizing a liquid contained therein through heating or ultrasonic vibration when a user smokes the electronic cigarette, so that the user can breathe in the smoke. A conventional electronic cigarette includes: a power supply part having a battery therein; and a smoke generating part containing a liquid therein and generating smoke by vaporizing the liquid using electricity supplied from the power supply part. Unlike typical cigarettes, the electronic cigarette having the above-mentioned construction does not produce harmful substances, such as tar, and does not produce bad odors, so that the electronic cigarette is widely used. However, the capacity of the battery of a conventional electronic cigarette is limited, so that most users of electronic cigarettes use portable charging devices for electronic cigarettes that are configured to hold the power supply part of an electronic cigarette therein and to charge the battery of the power supply part with electricity.

KR-Y-200451581 discloses a portable charging device for an electronic cigarette for charging a battery supplying electricity to an electronic cigarette, not providing additional functions other than the function of charging the battery of the electronic cigarette. Therefore, it is still impossible that a user smokes the electronic cigarette during a process of charging the battery of the power supply part with electricity using the charging device.

However, a conventional portable charging device for an electronic cigarette is problematic in that a user cannot smoke the electronic cigarette during a process of charging the battery of the power supply part with electricity using the charging device. Another problem of the conventional portable charging device for the electronic cigarette resides in that, to charge the battery of the electronic cigarette with electricity using the charging device, it is required to disassemble the power supply part from the smoke generating part and to set the power supply part in the charging device, so that it is inconvenient to users. Further, to charge the battery of the electronic cigarette with electricity using the portable charging device, the user should wait for a lengthy period of time although the conventional portable charging device does not provide additional functions other than the function of charging the battery of the electronic cigarette.

Accordingly, it is required to propose a portable charging device for an electronic cigarette that can provide additional functions of allowing a user to easily smoke the electronic cigarette while charging the battery of the electronic cigarette with electricity, and to sterilize the electronic cigarette while charging the battery of the electronic cigarette with electricity.

### Disclosure

### Technical Problem

Accordingly, the present invention has been made keeping in mind the above problems occurring in the related art, and an object of the present invention is to provide a portable charging device for an electronic cigarette, which can generate smoke while charging a battery of an electronic cigarette.

Another object of the present invention is to provide a portable charging device for an electronic cigarette, which can sterilize an electronic cigarette while charging a battery of an electronic cigarette.

A further object of the present invention is to provide a portable charging device for an electronic cigarette, which has a cap detachably attached to a main body such that the cap can cover an electronic cigarette set in the charging device so that the cap prevents the electronic cigarette from being contaminated with impurities, such as dust.

Still another object of the present invention is to provide a portable charging device for an electronic cigarette, which can easily charge a battery of an electronic cigarette without disassembling the electronic cigarette, thereby increasing using efficiency of the charging device.

Still another object of the present invention is to provide a portable charging device for an electronic cigarette, which can charge a battery of an electronic cigarette, can vaporize a liquid, and can supply electricity for operating a sterilization lamp using a single power source, thereby realizing compactness of a product.

Still another object of the present invention is to provide a portable charging device for an electronic cigarette, in which a reflective layer is formed in a cap and reflects ultraviolet rays radiated from a sterilization lamp, thereby maximizing the effect of sterilizing the electronic cigarette.

### Technical Solution

In order to accomplish the object, the present invention provides embodiments having the following constructions.

In an embodiment of the present invention, there is provided a portable charging device for an electronic cigarette, including: a main body having a first seat part electrically connected to a battery of an electronic cigarette, and a second seat part electrically connected to a smoke generating part of the electronic cigarette; and an operation unit electrically connected to both the first seat part and the second seat part, and controlling the charging of the battery and the operation of the smoke generating part, wherein the charging device can generate smoke from the smoke generating part while charging the battery of the electronic cigarette with electricity.

In another embodiment of the present invention, there is provided a portable charging device for an electronic cigarette, in which the first seat part may include: a first receiving chamber receiving a power supply part of the electronic cigarette therein, with the battery installed in the power supply part; and a first power supplying terminal provided in the first receiving chamber and transferring electricity from the operation unit to the power supply part, wherein, when the power supply part is inserted into the first receiving chamber, a charging terminal of the power supply part may come into contact with the first power supplying terminal, so that the battery may be electrically connected to the operation unit.

In a further embodiment of the present invention, there is provided a portable charging device for an electronic cigarette, in which the second seat part may include: a second receiving chamber receiving an end of the smoke generating part; and a second power supplying terminal provided in the second receiving chamber and transferring electricity from the operation unit to the smoke generating part, wherein, when the end of the smoke generating part is inserted into the second receiving chamber, a power receiving terminal of the smoke generating part may come into contact with the second power supplying terminal, so that the smoke generating part may be electrically connected to the operation unit.

In still another embodiment of the present invention, there is provided a portable charging device for an electronic cigarette, in which, when the end of the smoke generating part is inserted into the second receiving chamber, an inhaling bit of the smoke generating part may protrude outside the main body, so that the operation unit may be operated by sucking the inhaling bit held in a user's mouth and the smoke generating part may generate smoke, thereby allowing inhaling of the smoke.

In still another embodiment of the present invention, there is provided a portable charging device for an electronic cigarette, in which the operation unit may include: a cigarette charging module controlling the charging of the battery; and a smoke generating module controlling the smoke generating operation of the smoke generating part.

In still another embodiment of the present invention, there is provided a portable charging device for an electronic cigarette, in which the operation unit may further include: a power source supplying electricity for operating the charging device; and an input unit outputting signals for charging the battery and for starting the operation of the smoke generating part.

In still another embodiment of the present invention, there is provided a portable charging device for an electronic cigarette, further including: a cap detachably attached to the main body and receiving an inhaling bit of the smoke generating part therein.

In still another embodiment of the present invention, there is provided a portable charging device for an electronic cigarette, in which the main body may further include a power source terminal provided on a side surface of the main body and receiving electricity from the operation unit, and the cap may include: a power receiving terminal provided on a side surface of the cap, and a sterilization lamp electrically connected to the power receiving terminal and radiating ultraviolet rays, and sterilizing the electronic cigarette, wherein, when the cap is attached to the main body such that the inhaling bit is placed inside the cap, the power receiving terminal of the cap may come into contact with the power source terminal of the main body, so that the sterilization lamp may be electrically connected to the operation unit.

In still another embodiment of the present invention, there is provided a portable charging device for an electronic cigarette, in which the operation unit may include: a cigarette charging module controlling the charging of the battery; a smoke generating module controlling the smoke generating operation of the smoke generating part; and a sterilization module controlling an operation of the sterilization lamp.

### Advantageous Effects

The present invention will be provide the following advantages by a combination of the above-mentioned embodiments and the construction that will be described later herein.

The charging device of the present invention can sterilize an electronic cigarette while charging a battery of the electronic cigarette.

Further, the charging device of the present invention has a cap detachably attached to a main body such that the cap can cover an electronic cigarette set in the charging device so that the cap prevents the electronic cigarette from being contaminated with impurities, such as dust.

Further, the charging device of the present invention can easily charge the battery of the electronic cigarette without disassembling the electronic cigarette, thereby increasing using efficiency of the charging device.

Further, the charging device of the present invention can charge the battery of the electronic cigarette, can vaporize a liquid, and can supply electricity for operating a sterilization lamp using a single power source, thereby realizing compactness of a product.

Further, in the charging device of the present invention, a reflective layer is formed in the cap and reflects ultraviolet rays radiated from the sterilization lamp, thereby maximizing the effect of sterilizing the electronic cigarette.

### Description of Drawings

FIG. 1 is a perspective view of an electronic cigarette;
FIG. 2 is an exploded perspective view of the electronic cigarette;
FIG. 3 is a perspective view of a charging device according to an embodiment of the present invention;
FIG. 4 is a perspective view of the charging device according to the embodiment of the present invention, in which a cap is detached from a main body;
FIG. 5 is a sectional view of the charging device according to the embodiment of the present invention;
FIG. 6 is a bottom perspective view of the cap constituting the charging device according to the embodiment of the present invention;
FIG. 7 is a block diagram illustrating the construction of an operation unit constituting the charging device according to the embodiment of the present invention; and
FIG. 8 is a view illustrating an operation of the charging device according to the embodiment of the present invention.

### Best Mode

Hereinafter, a portable charging device for an electronic cigarette according to an embodiment of the present invention will be described with reference to the accompanying drawings. The same reference numerals throughout the drawings denote elements having the same or similar function. In the following description of the present invention, detailed descriptions of known functions and components incorporated herein will be omitted when it may make the subject matter of the present invention unclear. Unless otherwise defined, all terms including technical and scientific terms used herein have the same meaning as commonly understood by one of ordinary skill in the art to which this invention belongs. When terms used herein discord from the commonly understood meaning, the terms will be interpreted as defined herein.

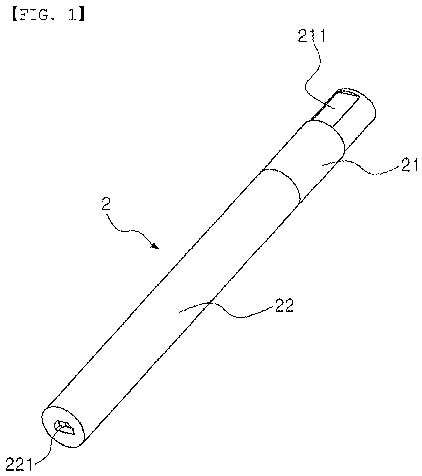

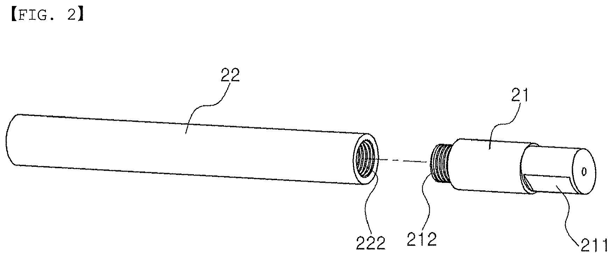

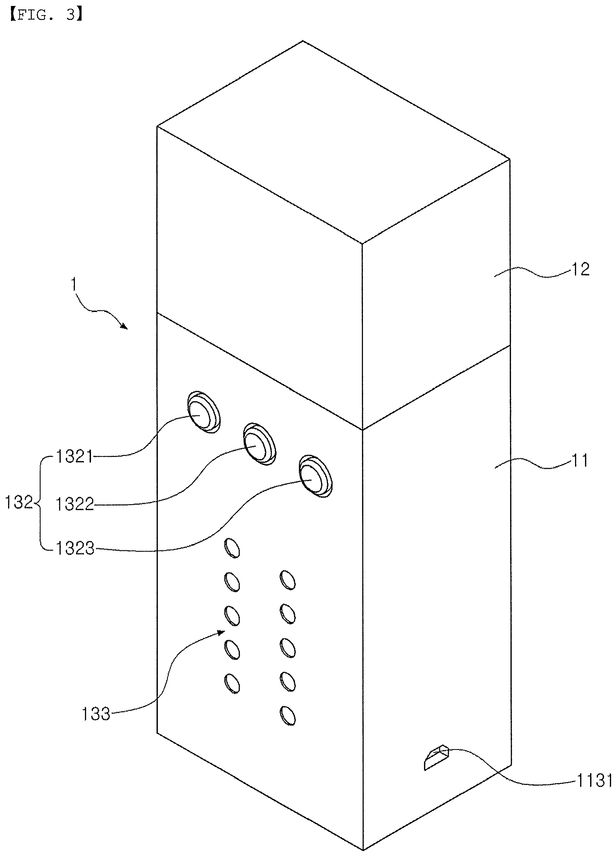

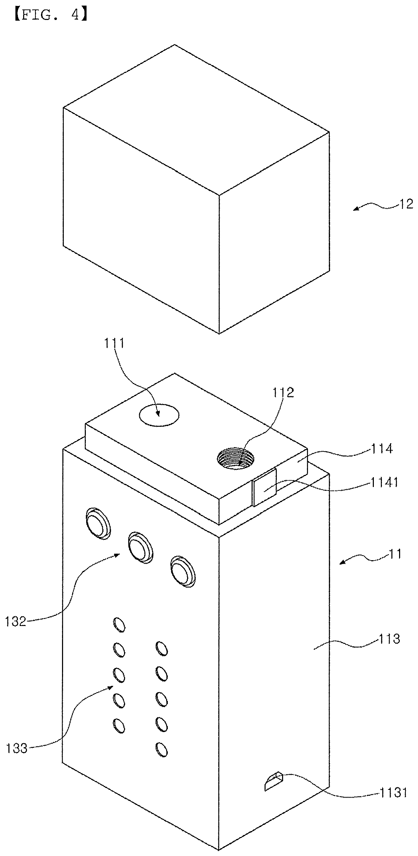

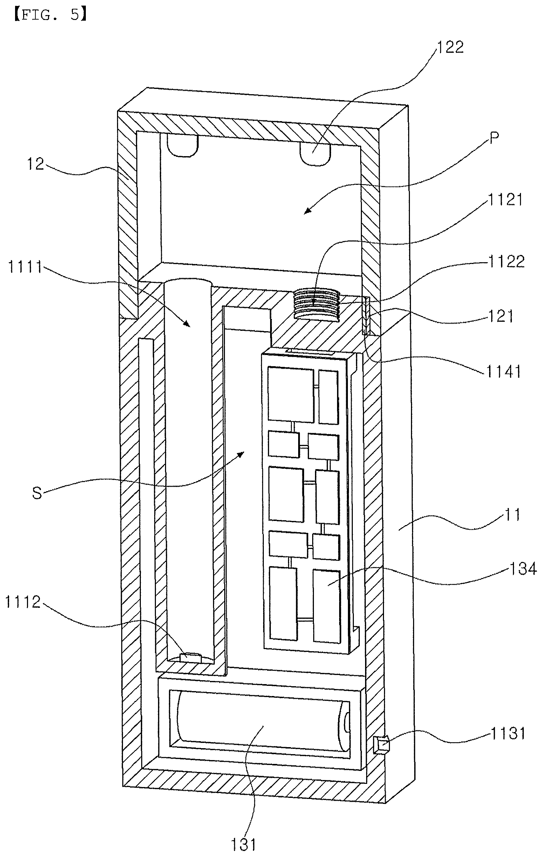

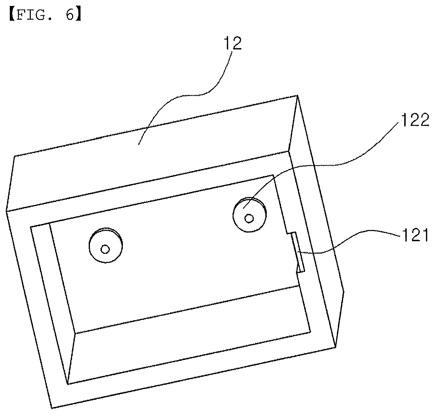

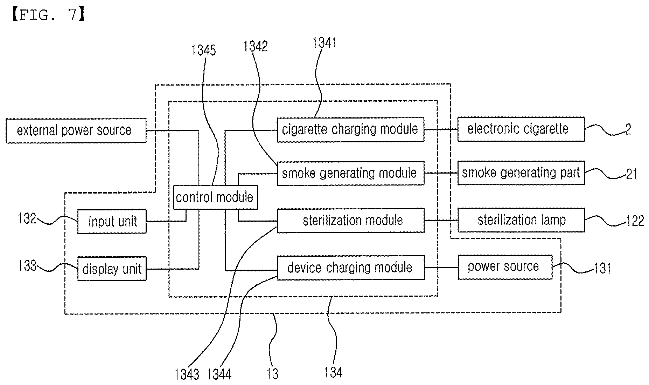

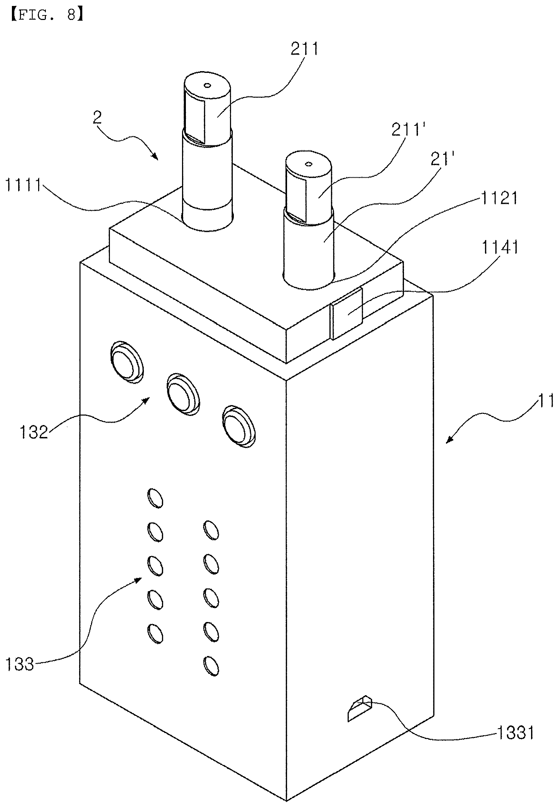

FIG. 1 is a perspective view of an electronic cigarette. FIG. 2 is an exploded perspective view of the electronic cigarette. FIG. 3 is a perspective view of a charging device according to an embodiment of the present invention. FIG. 4 is a perspective view of the charging device according to the embodiment of the present invention, in which a cap is detached from a main body. FIG. 5 is a sectional view of the charging device according to the embodiment of the present invention. FIG. 6 is a bottom perspective view of the cap constituting the charging device according to the embodiment of the present invention. FIG. 7 is a block diagram illustrating the construction of an operation unit constituting the charging device according to the embodiment of the present invention. FIG. 8 is a view illustrating an operation of the charging device according to the embodiment of the present invention.

A portable charging device for an electronic cigarette according to an embodiment of the present invention will be described with reference to FIGS. 1 to 8. The portable charging device 1 for an electronic cigarette of this invention includes: a main body 11 that receives an electronic cigarette 2, a cap 12 that is detachably attached to the main body 11 and sterilizes the electronic cigarette 2 placed therein; and an operation unit 13 that is installed in the main body 11 and controls the charging of the electronic cigarette 2, generation of smoke, and sterilization of the electronic cigarette 2. The charging device can generate smoke while charging a battery of the electronic cigarette 2, and can sterilize the electronic cigarette 2 during a process of charging the electronic cigarette 2.

Prior to describing the portable charging device 1 for the electronic cigarette, the construction of the electronic cigarette 2 will be described with reference to FIGS. 1 and 2. As shown in FIGS. 1 and 2, the electronic cigarette 2 includes: a smoke generating part 21 containing a liquid therein, and generating and emitting smoke by vaporizing the liquid using electricity supplied from a power supply part 22; and the power supply part 22 combined with the smoke generating part 21 and having the battery (not shown) therein so as to supply electricity to the smoke generating part 21. The smoke generating part 21 includes: an inhaling bit 211 that is formed on the upper end of the smoke generating part 21 and is held in a user's mouth when smoking using the electronic cigarette 2; and a power receiving terminal 212 that is spirally formed on the external circumferential surface of the lower end of the smoke generating part 21 and functions to receive electricity from the power supply part 22. The power supply part 22 includes: a charging terminal 221 that is formed in the lower end of the power supply part 22 and is connected to the charging device 1, and receives electricity from the charging device 1; and a power supplying terminal 222 that is spirally formed on the inner circumferential surface of the upper end of the power supply part 22 and supplies electricity to the power receiving terminal 212. Here, when the threaded lower end of the smoke generating part 21 is inserted into and fastened to the threaded upper end of the power supply part 22, the power supplying terminal 222 is engaged with the power receiving terminal 212, so that the smoke generating part 21 is electrically connected to the battery of the power supply part 22.

The main body 11 includes a first seat part 111 and a second seat part 112, forms the appearance of the charging device 1, and receives the electronic cigarette 2 therein. The main body 11 may have a predetermined shape. In an embodiment, the main body 11 may have a rectangular hexahedral shape formed by a lower part 113 and an upper part 114, in which the upper part 114 is provided on the lower part 113 and has a sectional area smaller than that of the lower part 113, with a receiving space S formed in the main body 11. Here, a charging terminal 1131 that receives electricity from an external power source is formed on a side surface of the lower part 113. A power source terminal 1141 that is electrically connected to the operation unit 13 and receives electricity from the operation unit 13 is provided on a side surface of the upper part 114. A controller 134 that will be described later herein is installed in the receiving space S.

The first seat part 111 includes a first receiving chamber 1111 and a first power supplying terminal 1112, and is electrically connected to the battery of the electronic cigarette 2.

The first receiving chamber 1111 is formed by being depressed downward from the upper end surface of the main body 11, and receives the power supply part 22 of the electronic cigarette 2 therein.

The first power supplying terminal 1112 is provided on a bottom surface of the first receiving chamber 1111, and transfers electricity from the operation unit 13 to the power supply part 22 of the electronic cigarette 2. In other words, when the power supply part 22 of the electronic cigarette 2 is set in the first receiving chamber 1111, the charging terminal 221 of the electronic cigarette 2 comes into contact with the first power supplying terminal 1112, so that the battery of the electronic cigarette 2 is electrically connected to the operation unit 13.

The second seat part 112 to which the smoke generating part 21 of the electronic cigarette 2 may be electrically connected includes a second receiving chamber 1121 and a second power supplying terminal 1122.

The second receiving chamber 1121 is formed by being depressed downward from the upper end surface of the main body 11, and receives the lower end of the smoke generating part 21 therein. The second receiving chamber 1121 may have a predetermined depth. In an embodiment, the depth of the second receiving chamber 1121 may be determined such that, when the lower end of the smoke generating part 21 is inserted into the second receiving chamber 1121, the inhaling bit 211 can be exposed outside the second receiving chamber 1121.

The second power supplying terminal 1122 is formed on the inner circumferential surface of the second receiving chamber 1121 and functions to transfer electricity from the operation unit 13 to the smoke generating part 21. In an embodiment, the second power supplying terminal 1122 may be formed as spiral threads made of a conductive material. When the threaded lower end of the smoke generating part 21 is inserted into and fastened to the second receiving chamber 1121, the power receiving terminal 212 of the smoke generating part 21 comes into contact with the second power supplying terminal 1122, so that the smoke generating part 21 is electrically connected to the operation unit 13.

The cap 12 is detachably attached to the main body 11 and receives the electronic cigarette 2 therein, and sterilizes the electronic cigarette 2. The cap 12 includes the power receiving terminal 121, a sterilization lamp 122, and a reflective layer (not shown). The cap 12 may have a predetermined shape. In an embodiment, the cap 12 may have a rectangular hexahedral shape in which the lower surface of the cap 12 is open. When the cap 12 is fitted over the upper part 114 of the main body 11, the cap 12 is attached to the main body 11 in which the lower edge of the cap 12 comes into contact with the upper end surface of the lower part 113 of the main body 11. When the cap 12 is attached to the main body 11 as described above, a receiving space P is defined inside the cap 12, so that the inhaling bit 211 protruding from the main body 11 can be received in the receiving space P.

The power receiving terminal 121 is formed on a lower part of the inner surface of the cap 12, so that, when the cap 12 is attached to the main body 11, the power receiving terminal 121 is electrically connected to the power source terminal 1141 and receives electricity from the power source terminal 1141.

The sterilization lamp 122 is electrically connected to the power receiving terminal 121, and radiates ultraviolet rays under the control of the operation unit 13, so that the sterilization lamp 122 can sterilize the electronic cigarette 2 placed in the receiving space P.

The reflective layer (not shown) is formed on the inner surface of the cap 12 and reflects ultraviolet rays radiated from the sterilization lamp 122, thereby increasing the effect of sterilizing the electronic cigarette 2 placed in the receiving space P of the cap 12 using the ultraviolet rays.

The operation unit 13 functions to charge the battery of the electronic cigarette 2 with electricity, and controls the operation of both the smoke generating part 21 and the sterilization lamp 122. The operation unit 13 includes a power source 131, an input unit 132, a display unit 133, a controller 134, etc.

The power source 131 supplies electricity for operating the charging device 1. For example, a lithium polymer rechargeable battery may be used as the power source 131. The power source 131 is electrically connected to both the controller 134 and the charging terminal 1131, and may be charged with electricity supplied from an external power source provided outside the charging device 1.

The input unit 132 is provided on the front surface of the main body 11 and functions to output signals for starting the charging of the electronic cigarette 2 and for starting the operation of both the smoke generating part 21 and the sterilization lamp 122 in response to a user's manipulation. The input unit 132 may include a push button 1321 for starting the charging of the battery of the electronic cigarette 2, a push button 1322 for starting the operation of the smoke generating part 21, and a push button 1323 for starting the operation of the sterilization lamp 122.

The display unit 133 is provided on the front surface of the main body 11 and displays the operation of the charging device 1. For example, LEDs may be used as the display unit 133 that can display the charging states of the charging device 1 and the electronic cigarette 2.

The controller 134 controls the charging of the electronic cigarette 2, and the operation of both the smoke generating part 21 and the sterilization lamp 122. The controller 134 may include a cigarette charging module 1341, a smoke generating module 1342, a sterilization module 1343, a device charging module 1344, a control module 1345, etc.

The cigarette charging module 1341 receives a signal indicative of starting the charging of the battery of the electronic cigarette 2 from the input unit 132, and supplies electricity to the first power supplying terminal 1112. Thus, the cigarette charging module 1341 charges the battery with electricity and displays the charged state of the battery on the display unit 133.

The smoke generating module 1342 receives a signal indicative of starting the operation of the smoke generating part 21 from the input unit 132, and controls a smoke generating operation of the smoke generating part 21.

The sterilization module 1343 operates the sterilization lamp 122 for a predetermined lengthy period of time in response to a signal output from the input unit 132 or to a signal indicative of a combination of the cap 12 with the main body 11.

The device charging module 1344 controls the charging of the power source 131 of the charging device 1.

The control module 1345 controls the operation of the cigarette charging module 1341, the smoke generating module 1342, the sterilization module 1343, and the device charging module 1344.

Hereinbelow, the operation of the portable charging device for the electronic cigarette 1 having the above-mentioned construction will be described with reference to FIGS. 1 to 8.

To charge the battery of the electronic cigarette 2 with electricity, the electronic cigarette 2 is inserted into the first receiving chamber 1111 such that the charging terminal 221 of the electronic cigarette 2 comes into contact with first power supplying terminal 1112. Thus, the battery of the electronic cigarette 2 is electrically connected to the operation unit 13. When a signal indicative of starting the operation of the battery is output in response to a user's manipulation on the input unit 132, the controller 134 supplies electricity to the battery, thus charging the battery with electricity and displaying the charged state of the battery on the display unit 133. The charging device 1 of the present invention can easily charge the battery of the electronic cigarette 2 without disassembling the electronic cigarette 2, thereby increasing using efficiency of the charging device.

To inhale smoke generated from the smoke generating part 21 of the electronic cigarette 2 using the charging device 1, a user inserts the smoke generating part 21 into the second receiving chamber 1121 such that the power receiving terminal 212 of the smoke generating part 21 comes into contact with the second power supplying terminal 1122. Accordingly, the smoke generating part 21 is electrically connected to the operation unit 13. Thereafter, the user manipulates the input unit 132 such that the input unit 132 outputs a signal for starting the operation of the smoke generating part 21. In response to the signal, the controller 134 supplies electricity to the smoke generating part 21. When the user inhales with the inhaling bit 211 in a user's mouth, the liquid contained in the smoke generating part 21 vaporizes and becomes smoke, so that the user can breathe in the smoke in a manner similar to that of directly smoking using the electronic cigarette 2. Although FIG. 8 illustrates that an electronic cigarette 2 having an smoke generating part 21 is set in the first receiving chamber 1111 and an additional smoke generating part 21' is set in the second receiving chamber 1121, the elements of one electronic cigarette 2 may be separately set in the two chambers 1111 and 1121 after disassembling the electronic cigarette 2. In other words, after disassembling one electronic cigarette 2, the smoke generating part 21 may be set in the second receiving chamber 1121 so as to generate smoke, and the power supply part 22 may be set in the first receiving chamber 1111 so as to charge the battery of the power supply part 22 with electricity.

To sterilize the electronic cigarette 2 using the charging device 1, the user inserts the electronic cigarette 2 into the first receiving chamber 1111, and inserts the additional smoke generating part 21' into the second receiving chamber 1121 such that both the inhaling bit 211 of the electronic cigarette 2 and the inhaling bit 211' of the additional smoke generating part 21' protrude outside the main body 11. Thereafter, the user attaches the cap 12 to the top of the main body 11 so that the two inhaling bits 221 and 221' are placed in the receiving space P. Further, the user manipulates the input unit 132 such that the input unit 132 outputs a signal for starting the operation of the sterilization lamp 122. In response to the signal, the controller 134 supplies electricity to the sterilization lamp 122 so that the sterilization lamp 122 radiates ultraviolet rays for a predetermined lengthy period of time, and sterilizes the inhaling bits 211 and 211' using the ultraviolet rays. Thus, the charging device 1 of the present invention is advantageous in that the charging device 1 can generate smoke from the smoke generating part 21 while charging the battery of the electronic cigarette 2 with electricity, or can sterilize the electronic cigarette 2 while charging the battery of the electronic cigarette 2 with electricity. Another advantage of the charging device 1 resides in that the charging device 1 can prevent the electronic cigarette 2 from being contaminated with impurities, such as dust, by holding the electronic cigarette 2 therein. A further advantage of the charging device 1 resides in that the charging device 1 can charge the battery of the electronic cigarette, can vaporize a liquid, and can supply electricity for operating the sterilization lamp using a single power source, thereby realizing compactness of a product.

Although the exemplary embodiments of the present invention have been described for illustrative purposes, those skilled in the art will appreciate that various modifications, additions and substitutions are possible, without departing from the scope and spirit of the present invention as disclosed in the accompanying claims.

## Claims

1. A portable charging device (1) for an electronic cigarette (2) for charging a battery supplying electricity to an electronic cigarette (2), the charging device (1) comprising:
a main body (11) having a first seat part (111) electrically connectable to a battery of an electronic cigarette (2) ; and
an operation unit (13) electrically connected to the first seat part (111) and controlling charging of the battery; **characterised in that** the main body (11) further includes a second seat part (112) electrically connectable to a smoke generating part (21) of the electronic cigarette (2), and wherein the operation unit (13) is electrically connected to the second seat part (112) and controls a smoke generating operation of the smoke generating part (21), so that the charging device (1) can generate smoke from the smoke generating part (21) while charging the battery of the electronic cigarette (2) with electricity.

2. The portable charging device (1) for the electronic cigarette (2) according to claim 1, wherein the first seat part (11) comprises:
a first receiving chamber (1111) receiving a power supply part (22) of the electronic cigarette (2) therein, with the battery installed in the power supply part (22); and
a first power supplying terminal (1112) provided in the first receiving chamber (1111) and transferring electricity from the operation unit (13) to the power supply part (22),
wherein, when the power supply part (22) is inserted into the first receiving chamber (1111), a charging terminal (221) of the power supply part (22) comes into contact with the first power supplying terminal (1112), so that the battery is electrically connected to the operation unit (13).

3. The portable charging device (1) for the electronic cigarette (2) according to claim 1, wherein the second seat part (112) comprises:
a second receiving chamber (1121) receiving an end of the smoke generating part (21); and
a second power supplying terminal (1122) provided in the second receiving chamber (1121) and transferring electricity from the operation unit (13) to the smoke generating part (21),
wherein, when the end of the smoke generating (21) part is inserted into the second receiving chamber (1121), a power receiving terminal (212) of the smoke generating part (21) comes into contact with the second power supplying terminal (1122), so that the smoke generating part (21) is electrically connected to the operation unit (13).

4. The portable charging device (1) for the electronic cigarette (2) according to claim 3, wherein, when the end of the smoke generating part (21) is inserted into the second receiving chamber (1121), an inhaling bit of the smoke generating part (21) protrudes outside the main body (11), so that the operation unit (13) can be operated by sucking the inhaling bit held in a user's mouth and the smoke generating part (21) can generate smoke, thereby allowing inhaling of the smoke.

5. The portable charging device (1) for the electronic cigarette (2) according to any one of claims 1 to 4, wherein the operation unit (13) comprises:
a cigarette charging module (1341) controlling the charging of the battery; and
a smoke generating module (1342) controlling the smoke generating operation of the smoke generating part (21).

6. The portable charging device (1) for the electronic cigarette (2) according to claim 5, wherein the operation unit (13) further comprises:
a power source (131) supplying electricity for operating the charging device (1); and
an input unit (132) outputting signals for charging the battery and for starting the operation of the smoke generating part (21).

7. The portable charging device (1) for the electronic cigarette (2) according to claim 1, further comprising:
a cap (12) detachably attached to the main body (11) and receiving an inhaling bit of the smoke generating part (21) therein.

8. The portable charging device (1) for the electronic cigarette (2) according to claim 7, wherein
the main body (11) further comprises a power source terminal (1141) provided on a side surface of the main body (11) and receiving electricity from the operation unit (13), and
the cap (12) comprises: a power receiving terminal (121) provided on a side surface of the cap (12); and a sterilization lamp (122) electrically connected to the power receiving terminal (121) and radiating ultraviolet rays, and sterilizing the electronic cigarette (2),
wherein, when the cap (12) is attached to the main body (11) such that the inhaling bit is placed inside the cap (12), the power receiving terminal (121) of the cap (12) comes into contact with the power source terminal (1141) of the main body (11), so that the sterilization lamp (122) is electrically connected to the operation unit (13).

9. The portable charging device (1) for the electronic cigarette (2) according to claim 8, wherein the operation unit (13) comprises:
a cigarette charging module (1341) controlling the charging of the battery;
a smoke generating module (1342) controlling the smoke generating operation of the smoke generating part (21); and
a sterilization module (1343) controlling an operation of the sterilization lamp (122).

## Patentansprüche

1. Tragbare Ladevorrichtung (1) für eine elektronische Zigarette (2) zum Laden einer Batterie, die Strom an eine elektronische Zigarette (2) liefert, wobei die Ladevorrichtung (1) Folgendes aufweist:
ein Grundkörper (11) mit einem ersten Auflageteil (111), das mit einer Batterie einer elektronischen Zigarette (2) elektrisch verbindbar ist; und
eine Bedieneinheit (13), die mit dem ersten Auflageteil (111) elektrisch verbunden ist und das Laden der Batterie steuert;
**dadurch gekennzeichnet, dass**
der Grundkörper (11) ferner ein zweites Auflageteil (112) umfasst, das mit einem Rauch erzeugenden Teil (21) der elektronischen Zigarette (2) elektrisch verbindbar ist, und wobei die Bedieneinheit (13) elektrisch mit dem zweiten Auflageteil (112) verbunden ist und einen Rauch erzeugenden Betrieb des Rauch erzeugenden Teils (21) steuert, sodass die Ladevorrichtung (1) Rauch von dem Rauch erzeugenden Teil (21) erzeugen kann, während die Batterie der elektronischen Zigarette (2) mit Strom geladen wird.

2. Tragbare Ladevorrichtung (1) für die elektronische Zigarette (2) gemäß Anspruch 1, wobei das erste Auflageteil (11) Folgendes aufweist:
eine erste Aufnahmekammer (1111), zur Aufnahme eines Energieversorgungsteil (22) der elektronischen Zigarette (2) darin, wobei die Batterie in dem Energieversorgungsteil (22) installiert ist; und
ein erster Energieversorgungsanschluss (1112), der in der ersten Aufnahmekammer (1111) vorgesehen ist und Strom von der Bedieneinheit (13) zu dem Energieversorgungsteil (22) überträgt,
wobei dann, wenn das Energieversorgungsteil (22) in die erste Aufnahmekammer (1111) eingeführt wird, ein Ladeanschluss (221) des Energieversorgungsteils (22) in Kontakt mit dem ersten Energieversorgungsanschluss (1112) kommt, sodass die Batterie elektrisch mit der Bedieneinheit (13) verbunden ist.

3. Tragbare Ladevorrichtung (1) für die elektronische Zigarette (2) gemäß Anspruch 1, wobei das zweite Auflageteil (112) Folgendes aufweist:
eine zweite Aufnahmekammer (1121) zur Aufnahme eines Ende des Rauch erzeugenden Teils (21); und
ein zweiter Energieversorgungsanschluss (1122), der in der zweiten Aufnahmekammer (1121) vorgesehen ist und Strom von der Bedieneinheit (13) zu dem Rauch erzeugenden Teil (21) überträgt,
wobei dann, wenn das Ende des Rauch erzeugenden Teils (21) in die zweite Aufnahmekammer (1121) eingeführt wird, ein Energieaufnahmeanschluss (212) des Rauch erzeugenden Teils (21) mit dem zweiten Energieversorgungsanschluss (1122) in Kontakt kommt, sodass das Rauch erzeugende Teil (21) elektrisch mit der Bedieneinheit (13) verbunden ist.

4. Tragbare Ladevorrichtung (1) für die elektronische Zigarette (2) gemäß Anspruch 3, wobei dann, wenn das Ende des Rauch erzeugenden Teils (21) in die zweite Aufnahmekammer (1121) eingeführt wird, ein Inhalationsstück des Rauch erzeugenden Teils (21) aus dem Grundkörper (11) heraus ragt, sodass die Bedieneinheit (13) durch Ansaugen des im Mund eines Benutzers gehaltenen Inhalationsstücks bedienbar ist und der Rauch erzeugende Teil (21) Rauch erzeugen kann, wodurch ein Inhalieren des Rauchs ermöglicht wird.

5. Tragbare Ladevorrichtung (1) für die elektronische Zigarette (2) gemäß einem der Ansprüche 1 bis 4, wobei die Bedieneinheit (13) Folgendes aufweist:
ein Zigarettenlademodul (1341), das die Aufladung der Batterie steuert; und
ein Rauch erzeugendes Modul (1342), das den Rauch erzeugenden Betrieb des Rauch erzeugenden Teils (21) steuert.

6. Tragbare Ladevorrichtung (1) für die elektronische Zigarette (2) gemäß Anspruch 5, wobei die Bedieneinheit (13) ferner Folgendes aufweist:
eine Energiequelle (131), die Strom zum Betreiben der Ladevorrichtung (1) liefert; und
eine Eingabeeinheit (132), die Signale zum Laden der Batterie und zum Starten der Bedienung des Rauch erzeugenden Teils (21) ausgibt.

7. Tragbare Ladevorrichtung (1) für die elektronische Zigarette (2) gemäß Anspruch 1, ferner Folgendes aufweisend:
eine Kappe (12), die lösbar an dem Grundkörper (11), zur Aufnahme eines Inhalationsstücks des Rauch erzeugenden Teils (21) darin, angebracht ist.

8. Tragbare Ladevorrichtung (1) für die elektronische Zigarette (2) gemäß Anspruch 7, wobei
der Grundkörper (11) ferner einen Energiequellenanschluss (1141) aufweist, der an einer Seitenfläche des Grundkörpers (11) vorgesehen ist und Strom von der Bedieneinheit (13) bezieht; und wobei
die Kappe (12) Folgendes aufweist: einen Energieaufnahmeanschluss (121), der auf einer Seitenfläche der Kappe (12) vorgesehen ist; und ein Sterilisationsleuchtmittel (122), das elektrisch mit dem Energieaufnahmeanschluss (121) verbunden ist und ultraviolette Strahlung ausstrahlt, und die elektronische Zigarette (2) sterilisiert,
wobei dann, wenn die Kappe (12) derart an dem Grundkörper (11) angebracht ist, dass das Inhalationsstück innerhalb der Kappe (12) angeordnet ist, der Energieaufnahmeanschluss (121) der Kappe (12) mit dem Energiequellenanschluss (1141) des Grundkörpers (11) in Kontakt kommt, sodass das Sterilisationsleuchtmittel (122) elektrisch mit der Bedieneinheit (13) verbunden ist.

9. Tragbare Ladevorrichtung (1) für die elektronische Zigarette (2) gemäß Anspruch 8, wobei die Bedieneinheit (13) Folgendes aufweist:
ein Zigarettenlademodul (1341), das die Aufladung der Batterie steuert;
ein Rauch erzeugendes Modul (1342), das den Rauch erzeugenden Betrieb des Rauch erzeugenden Teil (21) steuert; und
ein Sterilisationsmodul (1343), das einen Betrieb des Sterilisationsleuchtmittels (122) steuert.

## Revendications

1. Dispositif de chargement portable (1) pour une cigarette électronique (2), destiné à charger une batterie alimentant en électricité une cigarette électronique (2), le dispositif de chargement (1) comprenant :
un corps principal (11) ayant une première partie logement (111) pouvant être connectée électriquement à une batterie d'une cigarette électronique (2) ; et
un bloc fonctionnel (13) connecté électriquement à la première partie logement (111) et contrôlant le chargement de la batterie ;
**caractérisé en ce que** :
le corps principal (11) comprend en outre une seconde partie logement (112) pouvant être connectée électriquement à une partie de génération de fumée (21) de la cigarette électronique (2), le bloc fonctionnel (13) étant connecté électriquement à la seconde partie logement (112) et contrôlant une opération de génération de fumée de la partie de génération de fumée (21), de sorte que le dispositif de chargement (1) puisse générer de la fumée en provenance de la partie de génération de fumée (21) tout en chargeant en électricité la batterie de la cigarette électronique (2).

2. Dispositif de chargement portable (1) pour la cigarette électronique (2) selon la revendication 1, dans lequel la première partie logement (11) comprend :
une première chambre de réception (1111) recevant une partie alimentation (22) de la cigarette électronique (2), la batterie étant installée dans la partie alimentation (22); et
une première borne d'alimentation (1112) située dans la première chambre de réception (1111) et transférant de l'électricité du bloc fonctionnel (13) à la partie alimentation (22),
et dans lequel, quand la partie alimentation (22) est insérée dans la première chambre de réception (1111), une borne de chargement (221) de la partie alimentation (22) vient en contact avec la première borne d'alimentation (1112), de sorte que la batterie soit connectée électriquement au bloc fonctionnel (13).

3. Dispositif de chargement portable (1) pour la cigarette électronique (2) selon la revendication 1, dans lequel la seconde partie logement (112) comprend :
une seconde chambre de réception (1121) recevant une extrémité de la partie de génération de fumée (21); et
une seconde borne d'alimentation (1122) située dans la seconde chambre de réception (1121) et transférant de l'électricité du bloc fonctionnel (13) à la partie de génération de fumée (21),
et dans lequel, quand l'extrémité de la partie de génération de fumée (21) est insérée dans la seconde chambre de réception (1121), une borne de réception d'électricité (212) de la partie de génération de fumée (21) vient en contact avec la seconde borne d'alimentation (1122), de sorte que la partie de génération de fumée (21) soit connectée électriquement au bloc fonctionnel (13).

4. Dispositif de chargement portable (1) pour la cigarette électronique (2) selon la revendication 3, dans lequel, quand l'extrémité de la partie de génération de fumée (21) est insérée dans la seconde chambre de réception (1121), un embout d'inhalation de la partie de génération de fumée (21) fait saillie vers l'extérieur du corps principal (11), de sorte que le bloc fonctionnel (13) puisse être actionné en aspirant dans l'embout d'inhalation tenu dans la bouche d'un utilisateur, et que la partie de génération de fumée (21) puisse générer de la fumée, de manière à permettre l'inhalation de la fumée.

5. Dispositif de chargement portable (1) pour la cigarette électronique (2) selon l'une quelconque des revendications 1 à 4, dans lequel le bloc fonctionnel (13) comprend:
un module de chargement de cigarette (1341) contrôlant le chargement de la batterie ; et
un module de génération de fumée (1342) contrôlant l'opération de génération de fumée de la partie de génération de fumée (21).

6. Dispositif de chargement portable (1) pour la cigarette électronique (2) selon la revendication 5, dans lequel le bloc fonctionnel (13) comprend en outre :
une source électrique (131) fournissant de l'électricité pour le fonctionnement du dispositif de chargement (1); et
un bloc d'entrée (132) émettant des signaux pour charger la batterie et pour démarrer le fonctionnement de la partie de génération de fumée (21).

7. Dispositif de chargement portable (1) pour la cigarette électronique (2) selon la revendication 1, comprenant en outre :
un capuchon (12) fixé amovible au corps principal (11) et recevant un embout d'inhalation de la partie de génération de fumée (21).

8. Dispositif de chargement portable (1) pour la cigarette électronique (2) selon la revendication 7, dans lequel :
le corps principal (11) comprend en outre une borne de source électrique (1141) situé sur une surface latérale du corps principal (11) et recevant de l'électricité en provenance du bloc fonctionnel (13), et
le capuchon (12) comprend : une borne de réception d'électricité (121) située sur une surface latérale du capuchon (12) ; et une lampe de stérilisation (122) connectée électriquement à la borne de réception d'électricité (121) et émettant des rayons ultraviolets et stérilisant la cigarette électronique (2),
et dans lequel, quand le capuchon (12) est fixé au corps principal (11) de sorte que l'embout d'inhalation soit placé à l'intérieur du capuchon (12), la borne de réception d'électricité (121) du capuchon (12) vienne en contact avec la borne de source électrique (1141) du corps principal (11), de sorte que la lampe de stérilisation (122) soit connectée électriquement au bloc fonctionnel (13).

9. Dispositif de chargement portable (1) pour la cigarette électronique (2) selon la revendication 8, dans lequel le bloc fonctionnel (13) comprend :
un module de chargement de cigarette (1341) contrôlant le chargement de la batterie ;
un module de génération de fumée (1342) contrôlant l'opération de génération de fumée de la partie de génération de fumée (21); et
un module de stérilisation (1343) contrôlant un fonctionnement de la lampe de stérilisation (122).
